## Europäisches Patentamt

## European Patent Office

## Office européen des brevets

(11) Publication number : **0 374 105 B1**

(12) # EUROPEAN PATENT SPECIFICATION

(45) Date of publication of patent specification :
**19.05.93 Bulletin 93/20**

(51) Int. Cl.⁵ : **A61F 13/15**

(21) Application number : **89830509.9**

(22) Date of filing : **21.11.89**

(54) Absorbent element and absorbent article including the same.

(30) Priority : **25.11.88 IT 6805788**

(43) Date of publication of application :
**20.06.90 Bulletin 90/25**

(45) Publication of the grant of the patent :
**19.05.93 Bulletin 93/20**

(84) Designated Contracting States :
**AT BE CH DE ES FR GB GR IT LI LU NL SE**

(56) References cited :
**EP-A- 0 146 190
EP-A- 0 160 572
WO-A-87/05860
DE-B- 2 208 126
FR-A- 2 618 984
US-A- 4 413 995**

(73) Proprietor : **SOCIETA' CONSORTILE
RICERCHE ANGELINI S.p.A (or, briefly,
CONSORZIO RICERCHE S.p.A.)
Via Italica 101
I-65127 Pescara (IT)**

(72) Inventor : **Palumbo, Gianfranco
Via di Sotto 22
I-65125 Pescara (IT)**
Inventor : **Carlucci, Giovanni
Via Papa Giovanni XXIII, 65
I-66100 Chieti (IT)**
Inventor : **Di Girolamo, Remo
Via G. Bovio 413
I-65100 Pescara (IT)**

(74) Representative : **Bosotti, Luciano et al
c/o Jacobacci-Casetta & Perani S.p.A. Via
Alfieri, 17
I-10121 Torino (IT)**

## Description

### Field of the invention

The present invention relates to absorbent elements made from hydrophilic fibres, for examples cellulose fibres, in which the surface facing the user has a discontinuous layer of particles of an absorbent hydrogelling material.

In particular, the present invention relates to an improved absorbent element which can provide the user with an improved feeling of dryness by virtue of the presence of the discontinuous layer of hydrogelling particles.

### Description of the prior art

Disposable articles, such as babies' diapers, pads for incontinent adults, sanitary towels and similar products, are well known and are all provided with an absorbent element for absorbing and retaining body fluids.

Such absorbent element, commonly known as pad, must be able to take up the liquid rapidly and distribute it internally to avoid leakage at the sides; furthermore, it must have the capacity to retain the fluids when subjected to the normal pressures of use.

Absorbent pads made of cellulose fibres derived from conifer wood and bleached by a chemical process have satisfactory characteristics as regards the rate of absorption and distribution of the liquid, but are not very efficient from the point of view of retention and may allow liquid to flow back from the absorbent structure under the normal pressures of use.

One possible method of preventing this problem is to increase considerably the quantity of absorbent material in the pad so that the liquid does not flow back under the normal pressures of use; however, the fact that the cellulose is very hydrophilic means that in any event the surface of the absorbent pad tends to remain damp and can thus cause the user to feel wet.

An attempt has been made to resolve the liquid-backflow problem by mixing particles of absorbent hydrogelling material with the cellulose in order to increase the absorption and retention capacities of the absorbent element.

Use of such materials or materials having somewhat similar properties is widely known, as attested, e.g. by EP-A- 0 160 572, US-A- 4 413 995, WO-A- 8 705 860, or EP-A - 0 146 190.

For example, European Patent Application No. EP-A-0 122 042 proposes very dense absorbent structures constituted by a uniform mixture of particles of hydrogelling material and cellulose fibres.

The hydrogelling materials, commonly known as superabsorbents, are polymers which can absorb a large quantity of liquid, water or body fluids relative to their weight and can retain the fluids even under moderate pressure; because of these characteristics, their use in disposable absorbent articles has been proposed for some time.

Their good absorption capacity, however, is not matched by an equally good absorption rate and this can have an adverse effect on the performance of absorbent articles incorporating these substances.

In fact, superabsorbents can give rise to a phenomenon known as "gel blocking"; when superabsorbent particle comes into contact with the liquid, its outer surface starts to absorb and swells, obstructing the passage of the liquid into the particles; the liquid can only penetrate further into the still-dry core of the particles by means of a very slow diffusion mechanism.

This phenomenon not only prevents full use from being made of the large absorption capacities of superabsorbent substances, but can also involve a decrease in the absorption rate of structures with superabsorbent mixed with fibres, resulting in the possibility of lateral leakages.

Finally, although the superabsorbent substances have the advantage over cellulose fibres that they securely retain the liquid absorbed, the use of such substances simply mixed with cellulose fibres presents problems.

The many attempts of the prior art to resolve the problem have lead to the production of various devices using superabsorbents mixed with hydrophilic fibres.

European Patent Application No. EP-A-0 254 476 in the name of Procter and Gamble proposes a low-density zone positioned in the absorbent pad so as to receive the liquid directly upon its release: this zone constitutes a kind of temporary storage sump for the liquid which must then be absorbed into the surrounding denser zones of the pad.

A substantially different approach to the solution of the liquid-backflow problem from that based on the use of superabsorbent mixed with fibres is described in US Patent No. 3888256 in the name of H. Studinger.

In this solution, the superabsorbent is located only on the surface of a conventional pad; the quantity used

2

is such that, as the particles absorb and swell, they penetrate each other to form a continuous layer on top of the pad which prevents the liquid from flowing back under normal conditions of use; however, this layer also obstructs the absorption of further quantities of liquid which may be released subsequently.

There is thus a need for an absorbent element which rapidly absorbs and distributes the liquid, minimizing the risks of backflow and making adequate use of the characteristics of the absorbent hydrogelling substances.

## Objects and summary of the invention

More specifically the present invention relates to an absorbent element having the features set forth in the pre-characterising portion of Claim 1, which is known, e.g., from EP-A-0 146 190.

The object of the present invention is to avoid the problems described by virtue of an absorbent element of hydrophilic fibres having the further features called for in Claim 1.

Further advantages and characteristics of the invention form the subjects of the claims which follow the present description. In that respect the invention also refers to an absorbent article including the absorbent element of Claim 1.

## Detailed description of the invention

Further characteristics and advantages of the invention will become clear from the description which follows with reference to the appended drawings, provided by way of non-limiting example, in which:
- Figure 1 is a plan view of a disposable diaper produced according to the present invention;
- Figure 2 is a sectional view of the diaper taken on the longitudinal axis A-A' of Figure 1,
- Figure 3 is a plan view showing only the absorbent element of the diaper of Figure 1, two possible non-uniform distributions of the superabsorbent of which are shown in Figure 4 and 5;
- Figure 6 is a sectional view of a portion of the diaper incorporating the absorbent element of the present invention, on an enlarged scale;
- Figure 7 is a sectional view similar to that of Figure 6 but showing the absorbent element during the absorption of a certain quantity of liquid, and
- Figure 8 is yet another sectional view similar to that of Figure 6, showing the absorbent element during the absorption of a further quantity of liquid.

The absorbent elements of the present invention will be described with reference to their use in disposable absorbent articles; these articles are worn by the user in direct contact with the body, for the purpose of absorbing body fluids, and are subsequently thrown away after a single use.

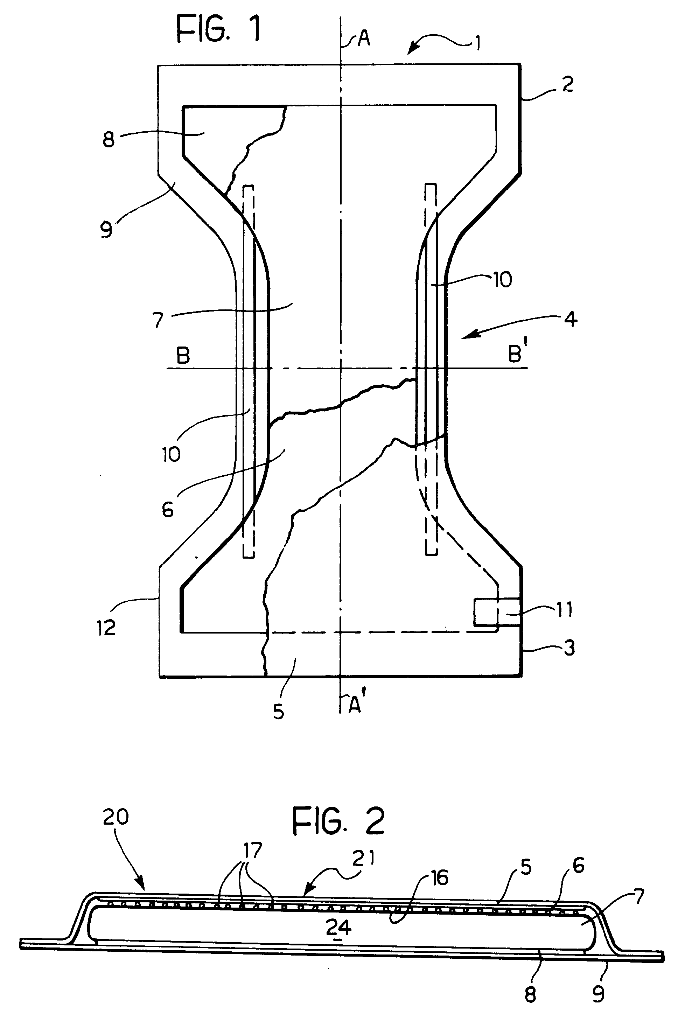

The disposable baby's diaper 1 shown in Figure 1 represents a preferred embodiment of an absorbent article according to the present invention, but the present invention is also intended to apply to other disposable absorbent articles, such as products for the incontinent, sanitary towels and the like.

Figure 1 is a plan view of a diaper in its stretched out configuration with some portions of the structure eliminated in order to show the construction of the diaper more clearly; in particular, the side of the diaper which comes into contact with the user is shown.

Figure 1 shows two end regions, a front region 2 and a rear region 3, which in use are positioned around the user's waist, and a central region 4 situated between them; a longitudinal axis AA' and a transverse axis BB' can also be seen.

The diaper includes an upper layer 5 of nonwoven fabric which is permeable to liquid and is intended to come into direct contact with the user's skin, a layer 6 of tissue immediately beneath the nonwoven fabric, an absorbent element 7, a second layer 8 of tissue, an impermeable plastics layer 9, and elastic elements 10 situated on either side of the absorbent element 7 in correspondence with its central region for effecting a seal around the legs of the user in use: one of the two adhesive tabs 11 necessary for fastening the diaper can also be seen on the rear region 3.

The outermost layers 5 and 9 of nonwoven fabric and plastics have the same shape and dimensions, corresponding to the outline 12 of the whole diaper, whilst the two tissue layers 6 and 8 are preferably shaped like the absorbent element 7 between them.

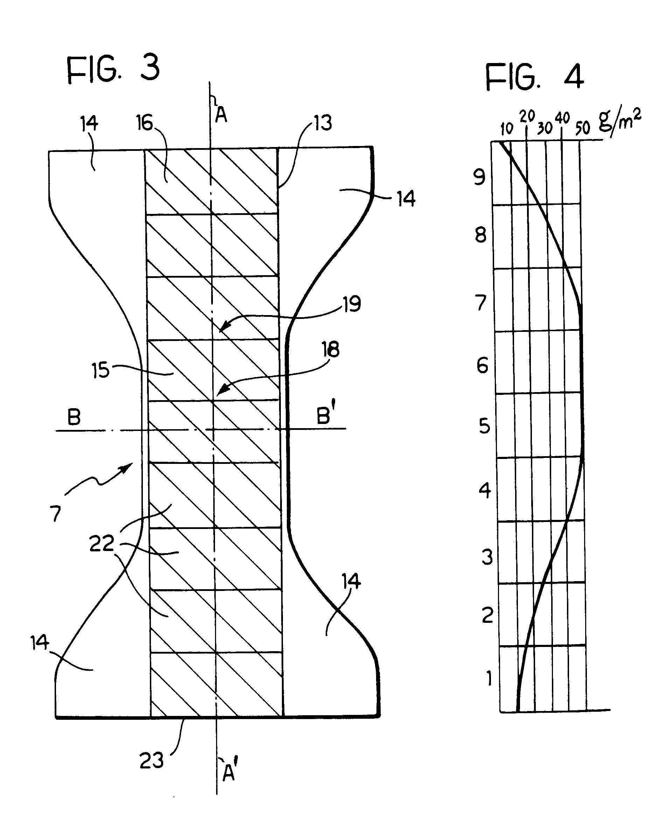

In the configuration shown, the absorbent element 7 is hourglass-shaped; in Figure 3, where it is shown on its own, it is easy to distinguish a central rectangular zone 13, with a length equal to the length of the element itself and a width approximately equal to its minimum width, and four peripheral zones 14, known as ears, situated on either side of the rectangular zone in correspondence with the two longitudinal ends of the absorbent element. The rectangular zone 13 includes the portion 15 of the absorbent element which is intended to receive the flow of liquid first since, in use, it is situated nearest the point from which the liquid is discharged; this zone can be referred to as a deposition zone.

The absorbent element 7 is composed solely of hydrophilic fibres 24, preferably of cellulose pulp.

Figure 2 shows that particles 17 of absorbent hydrogelling material are distributed non-continuously and non-uniformly on the upper surface 16 of the absorbent element 7, preferably only in correspondence with the rectangular central zone 13 shown in Figure 3.

Suitable absorbent hydrogelling substances may be inorganic or organic materials, such as cross-linked polymers wholly known in the art; preferably, the Chemische Fabrik Stockhausen superabsorbent product with the trade name Favor Sab 922 is used.

The average size of the particles 17, that is, the weighted average of the smallest dimension of the individual particles, may be between 100 and 800 microns, preferably between 200 and 500 microns.

The surface distribution of the superabsorbent particles 17 must be discontinuous, that is, such as to ensure that the particles are substantially isolated from each other before they absorb the liquid and that they remain so even after the swelling which results from the absorption, or, at any rate, that they do not form a continuous layer on the surface of the pad as they swell.

In general, this result is achieved with a surface distribution of particles 17 of the preferred dimensions at a density of between 5 $g/m^2$ and 70 $g/m^2$, preferably between 10 $g/m^2$ and 60 $g/m^2$.

In order to improve the performance of the absorbent element 7 of the present invention, the surface distribution of the superabsorbent is non-uniform, a large quantity being located in the deposition zone 15 of the pad, which has already been defined as the zone intended to receive the flow of liquid first since, in use, it is nearest to the point from which the liquid is discharged.

From an analysis carried out on normal diapers after use by plotting their absorption stains, it can be found that, because of the different anatomical forms of baby boys and baby girls, on average, the centre of the zone of the pad which first receives the flow of liquid may be located 55% of the way along the longitudinal axis AA' from the rear end, with respect to the total length of the pad, for girls and 65% of the way along for boys, as shown at 18 and 19 respectively in Figure 3.

The superabsorbent layer on the surface of the pad can thus be formed with a different non-uniform distribution for baby boys and baby girls which is particularly suitable for their different anatomical forms.

Although, in principle, the surface distribution of the superabsorbent may vary along both axes of the pad, the surface density of the particles 17 in a preferred embodiment depends solely on their position along the longitudinal axis AA' and, at any point on that axis, is constant for the whole width of the zone 13 where the superabsorbent 17 is deposited.

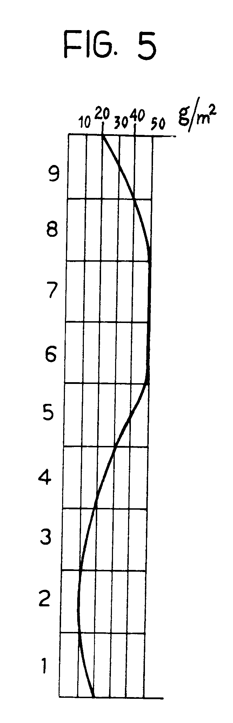

The surface distribution of the superabsorbent may vary gradually of discontinuously, along the longitudinal axis AA'; in the preferred configurations shown in Figures 4 and 5, the variation takes place along a continuous curve.

The extent of the portion 15 of the upper surface 16 of the pad 17 with a greater-than-average distribution of superabsorbent may be between 20% and 70% of the total surface of the zone with superabsorbent; the centre of the zone may be located on the longitudinal axis AA' of the pad at a distance of between 45% and 60% of the total length from the rear end 23 for diapers intended for girls and of between 60% and 70% of the total length for diapers intended for boys.

The centre of the zone with a greater surface density of superabsorbent preferably coincides with the centre of the deposition zone and is therefore located at points 55% (18) and 65% (19) of the way along the longitudinal axis AA' of the pad 7 for girls and for boys respectively.

Figure 2 shows that, compared with the average value of the surface density of the superabsorbent, there are zones 20 of lesser density and a zone 21 of greater density, corresponding to the deposition zone 15; the relative density values of the various zones must in any case fall within the lower and upper limits given above.

Figures 4 and 5 show the two preferred, non-uniform distributions of the superabsorbent particles 17 on the rectangular central zone 13 of the pad 7; this zone is shown divided into nine equal parts 22 along the longitudinal axis AA', whilst the graph of the right is a curve showing the distribution of the particles in $g/m^2$ as a function of the length of the zone.

Figure 4 shows the distribution suitable for girls' diapers: the centre 18 of the deposition zone 15 is on the longitudinal axis AA' at a distance of 55% of the total length of the pad from the rear end 23, with a maximum surface density of the superabsorbent of 50 $g/m^2$.

Figure 5, on the other hand, shows the distribution suitable for boys' diapers, which differs from the above in that the centre 19 of the deposition zone 15 is located 65% of the way along the total length of the pad 7 from the rear end 23.

In both cases, the deposition zone 15 represents approximately 55% of the upper surface of the pad on which the superabsorbent is distributed and which, in the configuration illustrated, corresponds to the central rectangular zone 13.

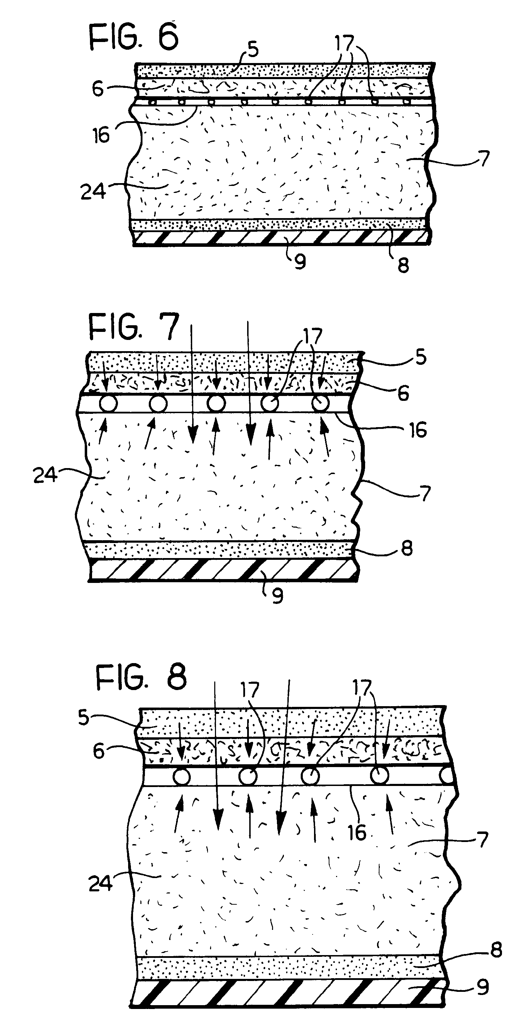

The way in which the absorbent structure described above functions can be shown by means of Figures 6 to 8: Figure 6 shows, in section, the initial configuration of a portion of the diaper.

At the moment when liquid is first released, the superabsorbent particles 17 start to swell slowly, while most of the liquid is absorbed by the fibres of the underlying pad 7: in Figure 7, the main flow of liquid into the absorbent element 7 is shown by the larger arrows.

As they swell, the particles 17 substantially do not come into contact with each other and aforementioned problem of the formation of a continuous layer of gelled superabsorbent which acts as a barrier to any subsequently released liquid is thus avoided; at the same time, the increase in volume of the particles 17 tends to lift the overlying layers of tissue 6 and nonwoven fabric 5 from the damp upper surface 16 of the pad 7.

The low absorption rate typical of the superabsorbent and the preferred, fairly large dimensions of the particles 17 mean that the particles are not saturated immediately but retain the capacity to absorb both the liquid still present in the layer of tissue 6, and possibly in the nonwoven fabric 5, and the liquid which may be squeezed out of the cellulose pad 7 and which tends to flow back to the surface because of the normal pressures to which the pad may be subjected in use; the secondary flows of liquid towards the particles 17, which are set up in use within the structure from the tissue 6, the nonwoven fabric 5 and the absorbent element 7 are represented by the smaller arrows in Figure 7.

The diaper 1 of the present invention can therefore provide the user with a greater feeling of dryness by virtue of the double effect of the superabsorbent particles 17 which, as they swell, tend to lift the tissue 6 and the nonwoven fabric 5 from the wet surface 16 of the pad 7, isolating it therefrom, and which, moreover, can still absorb any liquid which flows back from the pad 7 towards the outside under pressure since they are not completely saturated with liquid.

Moreover, the different non-uniform distribution of the superabsorbent diapers intended for boys and for girls provides a greater quantity of superabsorbent where it is most needed, that is, in correspondence with the deposition zones 15 which are situated differently for the two sexes.

The absorbent element 7 of the present invention has an improved capacity, even in the event of further releases of liquid by the user, as occurs, for example, when a diaper is worn through the night; Figure 8 shows that the superabsorbent particles 17 arranged discontinuously on the surface 16 of the pad remain separate from each other as they swell; the liquid can therefore always pass easily between the particles 17 towards the underlying pad 7 of cellulose fibres 24, as indicated by the larger arrows. The smaller arrows here also show the secondary flows of liquid towards the superabsorbent particles 17 which still retain a residual absorption capacity.

The absorbent element 7 of the present invention can therefore make full use of the characteristics of the material of which it is made: the cellulose fibres 24 confer speed of absorption and absorption capacity, whilst the superabsorbent particles 17 provide a retention capacity against the backflow of liquid; moreover, the non-uniform surface distribution of the superabsorbent, which is different in the diapers intended for the two sexes, contributed to the provision of an improved feeling of dryness for both boys and girls.

The behaviour of the diapers produced according to the present invention as regards the backflow of liquid under normal pressures of use has been verified by a series of laboratory tests using, for comparison, diapers with the same characteristics but without the superabsorbent.

All the diapers used had hourglass-shaped cellulose fibre pads with an average bulk of approximately 10 $cm^3/g$; the pads were 460 mm long, 290 mm wide at the front and rear ends, and 135 mm wide in the central region; the total weight of cellulose was 63 g.

The upper, liquid-permeable layer was of a continuous-thread, nonwoven fabric of hydrophobic polypropylene fibre about 0.180 mm thick and weighing approximately 23.00 $g/m^2$.

The intermediate layers placed between the nonwoven fabric layer and the absorbent element, and between said absorbent element and the plastics layer were made from a wet strength tissue about 0.080 mm thick and weighing about 19.00 $g/m^2$; the plastics layer was a liquid-impervious polyethylene film about 0.025 mm thick and weighing approximately 24.30 $g/m^2$.

The diapers with the superabsorbent were of the girls' type with and average quantity of superabsorbent on the entire surface of the central rectangular zone of the pad of approximately 35 $g/m^2$ distributed non-uniformly in the manner described: the deposition zone, characterised by a greater-than-average surface density of superabsorbent, was about 230 mm long; the highest density of superabsorbent, which was approximately 50 $g/m^2$, covered an area about 100 mm long in the deposition zone.

The centre of the deposition zone was situated 250 mm along the longitudinal axis of the pad from its rear end.

All the diapers were without elastics so that they could be laid flat.

The superabsorbent was Chemische Fabrik Stockhausen's Favor Sab 922, as already mentioned, in granules with an average size of between 200 and 500 microns.

A 1% solution of NaCl in distilled water was used as the liquid.

The test consisted of the pouring of 150 ml of solution onto the centre of the deposition zone of the diapers with superabsorbent on the surface and onto the centre of the pad of the control diapers without a deposition zone with distinguishing characteristics, over a period of 7 sec.

Then, 10 minutes, 20 min, 40 min, and 60 min after the administration of the liquid, the diapers were subjected to a single compression of 3.85 Kpa for a period of 3 sec, a previously-weighed sheet of absorbent paper of dimensions such as to cover the entire upper surface of the diaper having been interposed between the surface of the diaper and the weight; the sheet was white, absorbent, type "N" paper from Farini S.p.A.

As soon as the compression stage was completed, the sheet of absorbent paper was withdrawn and weighed again: the difference from the original weight corresponded to the quantity of liquid absorbed through the surface of the diaper, this being liquid which had been squeezed from the pad during the compression and had flowed back to the surface of the nonwoven fabric and which is responsible for the wet feeling noticed by the user in actual use.

The results of the test are summarised in the following table, where the values shown are the quantities of liquid absorbed by the paper, in grams:

| Type of diaper | Single compressions after a period of | | | |
|---|---|---|---|---|
| | 60' | 40' | 20' | 10' |
| Control diaper | 0.27 | 0.65 | 0.98 | 1.70 |
| Diaper according to the present invention | 0.00 | 0.00 | 0.01 | 0.09 |

As can be seen, compared to conventional diapers, the diapers provided with absorbent elements produced according to the present invention show a consistently improved behaviour as regards the prevention of the backflow of liquid.

## Claims

1. An absorbent element (7) made from hydrophilic fibres (24), wherein said absorbent element (7) has a discontinuous layer of absorbent hydrogelling material (17), characterised in that:
   - said layer (17) is distributed non-uniformly on the upper surface (16) of the element (7) which faces the user, and
   - - the element (7) includes a deposition zone (15) on its upper surface (16), and in that the absorbent hydrogelling material (17) is distributed on said deposition zone (15) with a surface density greater that the average value of the surface density.

2. An absorbent element (7) according to Claim 1, characterised in that the density of said absorbent hydrogelling material (17) distributed on the surface is between 5 $g/m^2$ and 70 $g/m^2$, preferably between 10 $g/m^2$ and 60 $g/m^2$.

3. An absorbent element (7) according to claim 1, characterised in that the centre of said deposition zone (15) is situated on the longitudinal axis (AA') of said absorbent element at a distance of between 45% and 70% of the total length of the pad from its rear end (23).

4. An absorbent element (7) according to any one of claims 1 or 3, characterised in that the extent of said deposition zone (15) is between 20% and 70% of the total surface (13) of the zone with the absorbent hydrogelling material.

5. An absorbent element (7) according to any one of the preceding claims, characterised in that the absorbent hydrogelling material (17) is preferably a polyacrylate.

6. An absorbent element (7) according to any one of the preceding claims, characterised in that the absorbent hydrogelling material (17) is in the form of particles with an average size of between 100 microns and 800 microns, preferably between 200 microns and 500 microns.

7. An absorbent article (1) including a lower, impermeable plastics sheet (9), an upper permeable sheet (5) of nonwoven fabric, and an absorbent element (7) positioned between the plastics sheet (9) and the nonwoven fabric sheet (5), characterised in that the absorbent element (7) is formed according to any one of the preceding claims.

8. An absorbent article (1) according to claim 7, in the form of a babies' diaper.

9. An absorbent article (1) according to any one of claims 7 or 8, characterised in that the entire (18) of said deposition zone (15) on the surface (16) of said absorbent element (7) is situated on the longitudinal axis (AA') of said absorbent element (7) at a distance of between 45% and 60% of the total length, preferably 55% of said length, from the rear end (23).

10. An absorbent article (1) according to any one of claims 7 or 8, characterised in that the centre (19) of said deposition zone (15) on the surface (16) of said absorbent element (7) is situated on the longitudinal axis (AA') of the absorbent element (7) at a distance of between 60% and 70% of the total length, preferably 65% of said length, from the rear end (23).

**Patentansprüche**

1. Absorbierendes Element (7) hergestellt aus hydrophilen Fasern (24), wobei dieses absorbierende Element (7) eine nicht durchgehende Schicht aus absorbierendem Hydrogelmaterial (17) aufweist, dadurch gekennzeichnet, daß die Schicht (17) auf der oberen, zum Benützer weisenden Fläche (16) des Elements (7) ungleichmäßig verteilt ist, und das Element (7) auf seiner oberen Fläche (16) einen Benetzungsbereich (15) umfaßt, und daß das absorbierende Hydrogelmaterial (17) auf dem Benetzungsbereich (15) mit einer Oberflächendichte verteilt ist, die größer ist als der Durchschnittswert der Oberflächendichte.

2. Absorbierendes Element (7) nach Anspruch 1, dadurch gekennzeichnet, daß die Dichte des absorbierenden, auf der Oberfläche verteilten Hydrogelmaterials (17) zwischen 5 g/m² und 70 g/m² beträgt, vorzugsweise zwischen 10 g/m² und 60g/m².

3. Absorbierendes Element (7) nach Anspruch 1, dadurch gekennzeichnet, daß das Zentrum des Benetzungsbereichs (15) auf der Längsachse (AA') des genannten absorbierenden Elements in einem Abstand von 45% bis 70% der Gesamtlänge des Elements von dessen hinterem Ende (23) entfernt liegt.

4. Absorbierendes Element (7) nach einem der Ansprüche 1 oder 3, dadurch gekennzeichnet, daß sich der Benetzungsbereich (15) über 20% bis 70% der Gesamtfläche (13) des Bereichs mit absorbierendem Hydrogelmaterial erstreckt.

5. Absorbierendes Element (7) nach einem der vorhergehenden Ansprüche, dadurch gekennzeichnet, daß das absorbierende Hydrogelmaterial (17) vorzugsweise ein Polyacrylat ist.

6. Absorbierendes Element (7) nach einem der vorhergehenden Ansprüche, dadurch gekennzeichnet, daß das absorbierende Hydrogelmaterial (17) die Form von Teilchen mit einer Durchschnittsgröße von 100 Mikrometer bis 800 Mikrometer, vorzugsweise von 200 Mikrometer bis 500 Mikrometer, hat.

7. Absorbierender Artikel (1) umfassend eine untere undurchlässige Kunststoffolie (9), eine obere durchlässige, dünne Schicht (5) aus Nonwoven und ein zwischen der Kunststoffolie (9) und der Nonwoven-Schicht (5) angeordnetes absorbierendes Element (7), dadurch gekennzeichnet, daß das absorbierende Element (7) gemäß einem der vorhergehenden Ansprüche beschaffen ist.

8. Absorbierender Artikel (1) nach Anspruch 7 in Form einer Babywindel.

9. Absorbierender Artikel (1) nach einem der Ansprüche 7 oder 8, dadurch gekennzeichnet, daß das Zentrum (18) des Benetzungsbereichs (15) auf der Oberfläche (16) des absorbierenden Elements (7) auf der Längsachse (AA') des absorbierenden Elements (7) in einem Abstand von 45% bis 60% der Gesamtlänge, vorzugsweise 55% dieser Länge, vom hinteren Ende (23) entfernt liegt.

10. Absorbierender Artikel (1) nach einem der Ansprüche 7 oder 8, dadurch gekennzeichnet, daß das Zentrum (19) des Benetzungsbereichs (15) auf der Oberfläche (16) des absorbierenden Elements (7) auf der Längsachse (AA') des absorbierenden Elements (7) in einem Abstand von 60% bis 70% der Gesamtlänge, vorzugsweise 65% dieser Länge, vom hinteren Ende (23) entfernt liegt.

## Revendications

1. Elément absorbant (7) fait de fibres hydrophiles (24), dans lequel ledit élément absorbant (7) comporte une couche discontinue de matériau hydrogélifiant absorbant (17), caractérisé en ce que ladite couche (17) est répartie de manière non uniforme sur la surface supérieure (16) de l'élément (7) qui fait face à l'utilisateur, et l'élément (7) comprend une zone réceptrice (15) sur sa surface supérieure (16), et en ce que le matériau hydrogélifiant absorbant (17) est réparti sur ladite zone réceptrice (15) avec une densité de surface plus grande que la valeur moyenne de la densité de surface.

2. Elément absorbant (7) selon la revendication 1, caractérisé en ce que la densité dudit matériau hydrogélifiant absorbant (17) réparti sur la surface est comprise entre 5 g/m$^2$ et 70 g/m$^2$, de préférence entre 10 g/m$^2$ et 60 g/m$^2$.

3. Elément absorbant (7) selon la revendication 1, caractérisé en ce que le centre de ladite zone réceptrice (15) est situé sur l'axe longitudinal (AA') dudit élément absorbant à une distance de 45 % à 70 % de la longueur totale de la garniture depuis son extrémité arrière (23).

4. Elément absorbant (7) selon l'une quelconque des revendications 1 ou 3, caractérisé en ce que l'étendue de ladite zone réceptrice (15) est comprise entre 20 % et 70 % de la surface totale (13) de la zone avec le matériau hydrogélifiant absorbant.

5. Elément absorbant (7) selon l'une quelconque des revendications précédentes, caractérisé en ce que le matériau hydrogélifiant absorbant (17) est, de préférence, un polyacrylate.

6. Elément absorbant (7) selon l'une quelconque des revendications précédentes, caractérisé en ce que le matériau hydrogélifiant absorbant (17) se présente sous la forme de particules d'une grosseur moyenne comprise entre 100 micromètres et 800 micromètres, de préférence entre 200 micromètres et 500 micromètres.

7. Article absorbant (1) comprenant une feuille de plastique imperméable, inférieure (9), une feuille perméable supérieure (5) en non-tissé, et un élément absorbant (7) positionné entre la feuille de plastique (9) et la feuille de non-tissé (5), caractérisé en ce que l'élément absorbant (7) est formé selon l'une quelconque des revendications précédentes.

8. Article absorbant (1) selon la revendication 7, se présentant sous la forme d'une couche pour bébé.

9. Article absorbant (1) selon l'une quelconque des revendications 7 ou 8, caractérisé en ce que le centre (18) de ladite zone réceptrice (15) sur la surface (16) dudit élément absorbant (7) est situé sur l'axe longitudinal (AA') dudit élément absorbant (7) à une distance de 45 % à 60 % de la longueur totale, de préférence de 55 % de ladite longueur, depuis l'extrémité arrière (23).

10. Article absorbant (1) selon l'une quelconque des revendications 7 ou 8, caractérisé en ce que le centre (19) de ladite zone réceptrice (15) sur la surface (16) dudit élément absorbant (7) est situé sur l'axe longitudinal (AA') de l'élément absorbant (7) à une distance de 60 % à 70 % de la longueur totale, de préférence de 65 % de ladite longueur, depuis l'extrémité arrière (23).

EP 0 374 105 B1

# FIG. 1

# FIG. 2

9

EP 0 374 105 B1

# FIG. 3

# FIG. 4

10

# FIG. 5

FIG. 6

FIG. 7

FIG. 8